(19) Europäisches Patentamt
European Patent Office
Office européen des brevets

(11) Veröffentlichungsnummer: **0 361 080 A1**

(12) **EUROPÄISCHE PATENTANMELDUNG**

(21) Anmeldenummer: **89115540.0**

(22) Anmeldetag: **23.08.89**

(51) Int. Cl.5: **C07C 43/13 , C07C 69/02 , C07D 303/16 , C07C 41/26**

(30) Priorität: **01.09.88 DE 3829743**

(43) Veröffentlichungstag der Anmeldung: **04.04.90 Patentblatt 90/14**

(84) Benannte Vertragsstaaten: **ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Stoll, Gerhard, Dr.**
**Danziger Strasse 69**
**D-4052 Korschenbroich 1(DE)**
Erfinder: **Fabry, Bernd, Dr.**
**Danziger Strasse 31**
**D-4052 Korschenbroich 1(DE)**

# (54) Verfahren zur Herstellung von Fettalkoholderivaten.

(57) Verfahren zur Herstellung von $C_{16}$-$C_{22}$-Fettalkoholderivaten der allgemeinen Formel I
$R^1$(vic. OH, $OR^2$) - OH (I)
in der
$R^1$ einen vicinal disubstituierten Rest eines Fettalkohols mit 16 bis 22 Kohlenstoffatomen, der mindestens ein Strukturelement der allgemeinen Formel II
- CH(OH) - CH ($OR^2$) - (II)
aufweist und
$R^2$ einen Alkylrest mit 1 bis 22 Kohlenstoffatomen, einen einbindigen Rest eines Polyols mit 2 bis 12 Kohlenstoffatomen und 2 bis 4 OH-Gruppen oder
eine Gruppe der allgemeinen Formel III

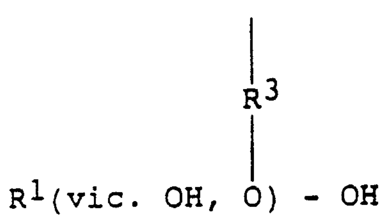

(III)

in der $R^3$ ein zweibindiger Rest einem Polyols mit 2 bis 12 Kohlenstoffatomen und 2 bis 4 OH-Gruppen, von denen mindestens 2 primäre OH-Gruppen sind, und $R^1$ wie oben definiert ist, bedeuten, lassen sich durch Behandlung mit Wasser unter Druck bei Temperaturen von 180 bis 250 °C aus $C_{16}$-$C_{22}$-Fettalkoholestern der Formel III bzw. IIIa
$R^1$(vic. OH, $OR^2$) - O - CO - $R^3$ (III)

EP 0 361 080 A1

$$R^1(\text{vic. OH, O}) - O - CO - R^3$$
$$|$$
$$R^{2'} \qquad \text{(IIIa)}$$
$$|$$
$$R^1(\text{vic. OH, O}) - O - CO - R^3$$

ohne Nebenreaktionen, insbesondere Selbstkondensationen, herstellen.

## Verfahren zur Herstellung von Fettalkoholderivaten.

Die Erfindung betrifft ein Verfahren zur Herstellung von Fettalkoholderivaten der allgemeinen Formel I

$R^1$(vic. OH, $OR^2$) - OH (I)

in der

$R^1$ einen vicinal disubstituierten Rest eines Fettalkohols mit 16 bis 22 Kohlenstoffatomen, der mindestens ein Strukturelement der allgemeinen Formel II

- CH(OH) - CH ($OR^2$) - (II)

aufweist und

$R^2$ einen Alkylrest mit 1 bis 22 Kohlenstoffatomen, einen einbindigen Rest eines Polyols mit 2 bis 12 Kohlenstoffatomen und 2 bis 4 OH-Gruppen oder eine Gruppe der allgemeinen Formel III

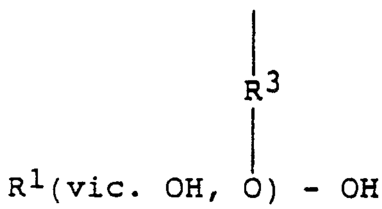

(III)

in der $R^3$ ein zweibindiger Rest einem Polyols mit 2 bis 12 Kohlenstoffatomen und 2 bis 4 OH-Gruppen, von denen mindestens 2 primäre OH-Gruppen sind, und $R^1$ wie oben definiert ist, bedeuten.

Die Herstellung von vicinal hydroxy, alkoxy- bzw. hydroxy, hydroxyalkylenoxy-substituierten $C_{16}$-$C_{22}$-Fettalkoholen, die verzweigte, nicht verseifbare Polyole darstellen, erfolgte bisher auf dem Wege über die Epoxidation der entsprechenden ungesättigten Fettalkohole mit anschließender Ringöffnung des Oxiran-Ringes mit einem ein- oder mehrwertigen Alkanol. Man erhielt auf diese Weise Ethergruppen enthaltende Polyole, deren OH-Funktionalität von der ursprünglichen Anzahl der Doppelbindungen, der Wertigkeit des zur Ringöffnung verwendeten Alkanols sowie dem Molverhältnis von Epoxyfettalkohol zu Alkanol bei der Ringöffnung abhängt, vgl. US-PS 2 491 533. Nachteilig bei diesem Verfahren ist die schwierige Herstellung von Epoxyfettalkoholen sowie der Umstand, daß die OH-Gruppe des Epoxyfettalkohols bei der Ringöffnung teilweise Produkte intra- und intermolekularer Kodensationsreaktionen ergibt.

Es wurde nun gefunden, daß die Titelverbindungen in besonders einfacher Weise und ohne nennenswerte Nebenreaktionen erhalten werden können, indem man $C_{16}$-$C_{22}$-Fettalkoholester der allgemeinen Formel IV bzw. IVa

$R^1$(vic. OH, $OR^2$) - O - CO - $R^4$ (IV)

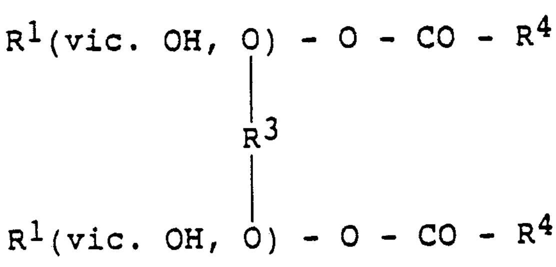

(IVa)

in der $R^4$ einen Alkylrest mit 1 bis 3 Kohlenstoffatomen bedeutet und $R^1$ (vic. OH, $OR^2$) sowie $R^2$ bzw. $R^3$ wie oben definiert sind, mit Wasser unter Druck bei Temperaturen von 180 bis 250 °C hydrolysiert.

Reste der die Gruppe $R^2$ bildenden Monoalkanole sind insbesondere die von geradkettigen oder verzweigten, gesättigten oder ungesättigten Alkanolen mit primären OH-Gruppen wie Methanol, Ethanol, Propanol, Butanol, Pentanol, Hexanol, Octanol, Decanol, Dodecanol, Tetradecanol, Hexadecanol, Oleylalkohol, Octadecanol, Behenylalkohol und Erucylalkohol einschließlich technischer Gemische derselben, wie sie in der Fettchemie üblicherweise eingesetzt werden.

Gemäß einer bevorzugten Ausführungsform des Verfahrens der Erfindung verwendet man einen Fettalkoholester der allgemeinen Formel IV bzw. IVa, in dem die Gruppe $R^2$ bzw. $R^3$ den Rest eines

Monoalkanols mit 1 bis 22 Kohlenstoffatomen oder eines mehrfunktionellen Alkanols mit mindestens einer primären OH-Gruppe aus der von Ethylenglykol, Diethylenglykol, Propylenglykol-1,2, Propylenglykol-1,3, Butandiol-1,4, Hexandiol-1,6, Decandiol-1,10, Dodecandiol-1,12, Glycerin, Diglycerin, Trimethylolpropan, Ditrimethylolpropan, Pentaerythrit und Dipentaerythrit gebildeten Gruppe bedeutet.

Die Herstellung der Titelverbindungen erfolgt bevorzugt nach einem neuen Gesamtverfahren, gemäß dem man die vicinal hydroxy, alkoxy bzw. hydroxy, hydroxyalkenyloxy substituierten $C_{16}$-$C_{22}$-Fettalkoholeester der allgemeinen Formel IV herstellt, indem man Fettalkohole der Formel $R^1$-OH, in der $R^1$ einen $C_{16}$-$C_{22}$-Alkenylrest mit einer olefinischen Doppelbindung oder mehreren ist, mit einem reaktiven Derivat einer $C_2$-$C_4$-Monocarbonsäure acyliert, die acylierten $C_{16}$-$C_{22}$-Fettalkohole epoxidiert und die Oxirangruppen der erhaltenen Epoxyfettalkoholester mit Monoalkanolen mit 1 bis 22 Kohlenstoffatomen bzw. mehrfunktionellen Alkanolen mit 2 bis 12 Kohlenstoffatomen und 2 bis 4 OH-Gruppen in Gegenwart katalytisch wirkender Lewissäuren umsetzt, und die erhaltenen Verbindungen der Formel IV bzw. IVa in die Titelverbindungen überführt.

Als reaktive Derivate der $C_2$-$C_4$-Monocarbonsäuren eignen sich z.B. Ester und Säurehalogenide, insbesondere jedoch die Anhydride.

Bevorzugt ist die Gruppe $R^1$ eine lineare Alkenylgruppe mit 16 bis 22 Kohlenstoffatomen, die eine oder mehr als eine olefinische Doppelbindungen aufweisen kann, insbesondere eine Fettalkylgruppe, die überwiegend aus Oleyl-, cis-6-Octadecenyl-, Elaidyl-, Palmitoleyl-, Linoleyl-, Linolenyl-, Gadoleyl- und/oder Erucylgruppen besteht. Derartige Fettalkohole sind aus synthetischen oder natürlichen Rohstoffen, z.B. Rindertalg, Schweineschmalz oder pflanzlichen Ölen wie Sonnenblumen-, Koriander-, Soja- oder Rapsöl herstellbar und stellen im allgemeinen technische Gemische verschiedener Fettalkohole dar. Der Monocarbonsäurerest der intermediär hergestellten acylierten $C_{16}$-$C_{22}$-Fettalkohole ist von einer Monocarbonsäure mit 2 bis 4 Kohlenstoffatomen, z.B. Propionsäure oder Butancarbonsäure, bevorzugt jedoch Essigsäure, abgeleitet.

Das Gesamtverfahren wird bevorzugt mit Fettalkoholen der Formel $R^1$-OH bzw. Fettalkoholgemischen durchgeführt, die Jodzahlen von 55 und darüber aufweisen; im Falle mehrfach ungesättigter Fettalkohole kann man gegebenenfalls mehrere der vorhandenen olefinischen Doppelbindungen epoxidieren und kommt letztlich zu hydroxy, alkoxy bzw. hydroxy, hydroxyalkylenoxy-substituierten $C_{16}$-$C_{22}$-Fettalkoholen, die mehr als die Mindestzahl von 2 Hydroxylgruppen pro Molelül enthalten, d.h. die mehr als ein Strukturelement der Formel II aufweisen.

Die acylierten $C_{16}$-$C_{22}$-Fettalkohole können in an sich bekannter Weise epoxidiert werden, z.B. mit Persäuren, insbesondere mit in situ hergestellter Perameisensäure. Die dabei erhaltenen Epoxyfettalkoholeester werden mit Monoalkanolen mit 1 bis 22 Kohlenstoffatomen bzw. mehrfunktionellen Alkanolen mit 2 bis 12 Kohlenstoffatomen und 2 bis 4 OH-Gruppen in Gegenwart katalytisch wirkender Lewissäuren umgesetzt; typische Beispiele für derartige Lewissäuren sind Schwefelsäure, Methansulfonsäure, Toluolsulfonsäure sowie saure Ionenaustauscher. Die Umsetzung erfolgt möglichst wasserfrei bei Molverhältnissen von Epoxyfettsäureester zu mono- bzw. mehrfunktionellen Alkanolen von etwa 2:1 bis 1:6.

Bei Einsatzmolverhältnissen von Epoxyfettsäureestern zu mehrfunktionellen Alkanolen von etwa 2:1 entstehen durch intramolekulare Kondensation überwiegend der Einfachheit halber hier und im folgenden als "dimer" bezeichnete Fettsäureester der Formel IVa, die als Endprodukte des Verfahrens der Erfindung die entsprechenden "dimeren" Fettalkohole der Formel

$$\begin{array}{c} R^1(\text{vic. OH, O}) - OH \\ | \\ R^3 \\ | \\ R^1(\text{vic. OH, O}) - OH \end{array}$$

ergeben.

"Dimere" Fettalkohole entstehen z.B. vorwiegend bei der Umsetzung mit Glycerin oder Diglycerin, da sekundäre OH-Gruppen reaktionsträge sind. Besonders bevorzugt sind hier difunktionelle lineare Alkanole mit 2 bis 12 Kohlenstoffatomen.

Mit Einsatzmolverhältnissen von etwa 1:1 bis 1:6 entstehen mit mehrfunktionellen Alkanolen überwiegend "monomere" Fettsäureester bzw. als Endprodukte die "monomeren" Fettalkohole.

Die Reaktionsprodukte können nach der Umsetzung mit wasserfreien Basen, z.B. Alkali- oder Erdakalioxiden, -hydroxiden oder -carbonaten, Alkalialkoholaten, Erdalkalialkoholaten oder Aminen, neutralisiert werden; anschließend erfolgt die Entfernung des überschüssigen ein-oder mehrfunktionellen Alkanols, insbesondere durch Destillation oder Phasenverteilung.

Die so erhaltenen Fettalkoholester der allgemeinen Formel IV bzw. IVa werden anschließend, vorzugsweise ohne Katalysatoren und in Abwesenheit von organischen Hilfslösemitteln, mit Wasser unter Druck bei den angegebenen Temperaturen hydrolysiert. Bevorzugt wird dabei ein Volumenverhältnis von Fettalkoholester der allgemeinen Formel IV bzw. IVa zu Wasser von 1:0,5 bis 1:6, insbesondere 1:0,8 bis 1:1,5, angewendet. Die Druckspaltung kann in einem Autoklaven oder auch in einem Säulenreaktor erfolgen.

Die Erfindung wird im folgenden anhand von bevorzugten Beispielen näher erläutert.

Beispiel 1.

Herstellung eines vicinal hydroxy, hydroxyethylenoxysubstituierten Stearylalkohols aus Oleylalkohol.

Oleylacetat (technisch).

Ein technischer Oleylalkohol (Jodzahl 94, Hydroxylzahl 210) wurde mit Essigsäureanhydrid (20 mol Überschuß) bei 118 °C 4 Stunden umgesetzt. Das Reaktionsgemisch wurde auf Eiswasser gegossen; die organische Phase wurde mehrmals mit Wasser gewaschen. Anschließend wurde der erhaltene Rohester getrocknet und durch Destillation gereinigt. Der erhaltene Ester wies eine Jodzahl von 83 und eine Resthydroxylzahl von 0,9 auf.

Epoxidation von Oleylacetat.

Zu einer Mischung von 610,1 g (2 mol) Oleylacetat, erhalten gemäß der obigen Arbeitsvorschrift, und 29,5 g (0,36 mol) 85 %-iger Ameisensäure wurden innerhalb von 20 min bei 50 bis 62 °C 136 g (2,8 mol) 70 %-iges Wasserstoffperoxid getropft. Nach 7 3/4 h Rühren bei 60 °C wurde die wässrige Phase abgetrennt und die organische Phase dreimal mit je 1 l warmem Wasser neutral gewaschen sowie schließlich im Vakuum getrocknet. Man erhielt 623 g einer klaren, farblosen Flüssigkeit.

Analytische Daten:

Epoxid-O-Gehalt: 4,00 % (theor.: 4,98)
Verseifungszahl: 183,2
Jodzahl: 4,8
Säurezahl: 0,5.

Ringöffnung des epoxidierten Oleylacetats mit Ethylenglykol.

921,6 g (2,35 mol) epoxidiertes Oleylacetat, erhalten nach der obigen Arbeitsvorschrift, 437,6 g (7,5 mol) Ethylenglykol und 0,3 g konzentrierte Schwefelsäure wurden 40 min bei 100 bis 110 °C gerührt. Nach Neutralisation der Schwefelsäure mit 2,0 g Natriummethylat wurde im Vakuum bis 130 °C zur Entfernung des Ethylenglykols destilliert. Man erhielt eine klare, farblose Flüssigkeit.

Analytische Daten:

Hydroxylzahl: 245,1 (theor.: 247)
Verseifungszahl: 149,7 (theor.: 158,5)
Säurezahl: 0,3.

Druckspaltung des vicinal hydroxy, hydroxyethylenoxysubstituierten Stearylacetats.

100 g Ringöffnungsprodukt des epoxidierten Oleylacetats, hergestellt nach vorstehender Arbeitsvorschrift, wurde dreimal mit je 100 ml Wasser im Autoklaven auf 250 °C erhitzt. Nach dem Trocknen erhielt man eine braune, klare, viskose Flüssigkeit.

Analytische Daten:

Hydroxylzahl: 405,5
Verseifungszahl: 12,9
Jodzahl: 8,4
Säurezahl: 1,3

Beispiel 2.

Herstellung eines vicinal hydroxy, hydroxyethylenoxysubstituierten Stearylalkohols aus Oleylalkohol ex Rüböl.

Rübocenolacetat (technisch).

464,1 g (1,7 mol) technischer Oleylalkohol ex Rüböl (Jodzahl 99, Hydroxylzahl 205,5,) wurde mit 208,1 g (2,04 mol) Essigsäureanhydrid 8 h unter Rückfluß gekocht. Das überschüssige Anhydrid wurde im Wasserstrahlvakuum abdestilliert, der Rückstand mit festem Natriumcarbonat unter Rühren neutralisiert (SZ < 1), mit Natriumsulfat getrocknet und nach Entfernen der Salze durch Filtration im Vakuum destilliert. Der so erhaltene Ester wies eine Jodzahl von 85,9 und eine Resthydroxylzahl von 0,3 auf.

Epoxidation von Rübocenolacetat.

Zu einer Mischung von 163,4 g (0,55 mol) Rübocenolacetat, erhalten gemäß der obigen Arbeitsvorschrift, und 5,4 g (0,01 mol) 85%-iger Ameisensäure wurden innerhalb von 30 min bei 65 bis 70 °C 37,6 g (0,77 mol) 70%iges Wasserstoffperoxid getropft. Nach 4 3/4 h Rühren bei 70 °C wurde die wäßrige Phase abgetrennt und die organische Phase dreimal mit je 100 ml Wasser neutral gewaschen sowie schließlich im Vakuum getrocknet. Man erhielt so 176,4 g einer farblosen, klaren Flüssigkeit.

Analytische Daten:

Epoxid-O-Gehalt: 4,15 (theor.:5,13)
Verseifungszahl: 180,2
Jodzahl: 7,0
Säurezahl: 0,2

Ringöffnung des epoxidierten Rübocenolacetats mit Ethylenglykol.

136,5 g (0,35 mol) epoxidiertes Rübocenolacetat, erhalten nach obiger Arbeitsvorschrift, 66 g (1,06 mol) Ethylenglykol und 0,035 g (1,0 g/mol) konzentrierte Schwefelsäure wurden 45 min bei 100 bis 110 °C gerührt. Nach Neutralisation der Schwefelsäure mit 0,13 g Natriummethylat wurde im Vakuum bis 130 °C zur Entfernung des überschüssigen Ethylenglykols destilliert. Man erhielt so eine klare, hellgelbe Flüssigkeit.

Analytische Daten:

Hydroxylzahl: 245% (theor.: 251)
Verseifungszahl: 152,2 (theor.: 155)
Säurezahl: 0,5

Jodzahl: 7,7

Druckspaltung des vicinal hydroxy, hydroxyethylenoxysubstituierten Rübocenolacetats.

130 g Ringöffnungsprodukt des epoxidierten Rübocenolacetats, hergestellt nach vorstehender Arbeitsvorschrift wurden mit 780 g Wasser 6 h unter autogenem Druck (22 bar) im Autoklaven auf 220 °C erhitzt. Nach dem Trocknen erhielt man 103 g einer klaren, braunen Flüssigkeit.

Analytische Daten:

Hydroxylzahl: 355,4 (theor.:460)
Verseifungszahl: 57,3
Säurezahl: 1,7
Jodzahl: 14,6
Wasserhalt: 0,10% nach Fischer
Viskosität: 774 mPas (nach Höppler/20 °C)

Beispiel 3.

Herstellung eines "dimeren", vicinal hydroxy, ethylendioxy-substituierten Stearylalkohols.

Aus 2 Mol des in Beispiel 1 beschriebenen epoxidierten Oleylacetats läßt sich bei der Ringöffnung mit 1 Mol Ethylenglykol und anschließender Druckspaltung analog zu der in Beispiel 1 beschriebenen Weise die Titelverbindung erhalten, die einen tetrafunktionellen Alkohol darstellt.

Beispiel 4.

Herstellung eines vicinal hydroxy, methoxy-substituierten Stearylalkohols.

394,1 g (1 Mol) eines epoxidierten Oleylacetats (Epoxid-O-Gehalt 4,06%), erhalten nach der in Beispiel 1 angegebenen Arbeitsvorschrift, 192 g (6 Mol) Methanol und 0,6 g konzentrierte Schwefelsäure wurden 5,5 Stunden unter Rückfluß gerührt. Nachdem der Epoxid-O-Gehalt auf 0 abgesunken war, wurde mit 2,6 g einer 30%-igen methanolischen Natriummethylatlösung neutralisiert; das überschüssige Methanol wurde im Vakuum (15 Torr) bei 100 °C entfernt. Nach Filtration erhielt man eine klare, gelbe Flüssigkeit.

Analytische Daten:

Hydroxylzahl 154,2 (theor. 132)
Verseifungszahl 160,5 (theor. 170,5)
Jodzahl 4,6
Säurezahl 0,7.

Druckspaltung des vicinal hydroxy, methoxy-substituierten Stearylacetats.

130 g des oben hergestellten Ringöffnungsprodukts wurden mit 780 g Wasser 6 Stunden bei 250 °C unter autogenem Druck (40 bar) gerührt. Nach Abtrennen des Wassers und Trocknen der organischen Phase erhielt man eine klare, braune Flüssigkeit.

Analytische Daten:

Hydroxylzahl 253,2 (theor. 337)
Verseifungszahl 63,0

Säurezahl 1,2
Jodzahl 9,8.

## Ansprüche

1. Verfahren zur Herstellung von Fettalkoholderivaten der allgemeinen Formel I
$R^1$(vic. OH, $OR^2$) - OH (I)
in der
$R^1$ einen vicinal disubstituierten Rest eines Fettalkohols mit 16 bis 22 Kohlenstoffatomen, der mindestens ein Strukturelement der allgemeinen Formel II
- CH(OH) - CH ($OR^2$) - (II)
aufweist und
$R^2$ einen Alkylrest mit 1 bis 22 Kohlenstoffatomen, einen einbindigen Rest eines Polyols mit 2 bis 12 Kohlenstoffatomen und 2 bis 4 OH-Gruppen oder
eine Gruppe der allgemeinen Formel III

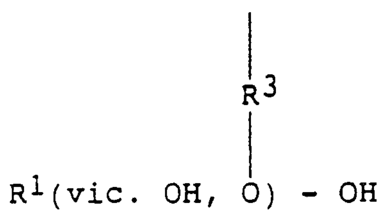

(III)

in der $R^3$ ein zweibindiger Rest einem Polyols mit 2 bis 12 Kohlenstoffatomen und 2 bis 4 OH-Gruppen, von denen mindestens 2 primäre OH-Gruppen sind, und $R^1$ wie oben definiert ist, bedeuten, dadurch gekennzeichnet, daß man vicinal hydroxy, alkohxy- bzw. hydroxy, hydroxyalkylenoxy-substituierte $C_{16}$-$C_{22}$-Fettalkoholester der allgemeinen Formel IV bzw. IVa
$R^1$(vic. OH, $OR^2$) - O - CO - $R^4$ (IV)

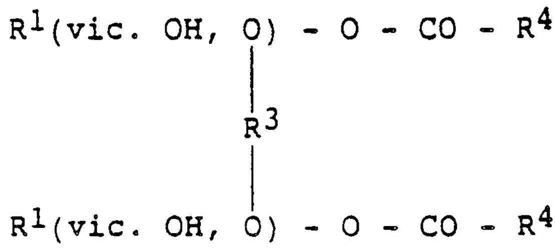

(IVa)

in der $R^4$ einen Alkylrest mit 1 bis 3 Kohlenstoffatomen bedeutet und $R^1$(vic. OH, $OR^2$) sowie $R^2$ bzw. $R^3$ wie oben definiert sind, mit Wasser unter Druck bei Temperaturen von 180 bis 250 °C hydrolysiert.

2. Verfahren nach Anspruch 1, dadurch gekennzeichnet, daß man einen Fettalkoholester der allgemeinen Formel IV bzw. IVa verwendet, in der die Gruppe $R^2$ bzw. $R^3$ den Rest eines mehrfunktionellen Alkanols aus der von Ethylenglykol, Diethylenglykol, Propylenglykol-1,2, Propylenglykol-1,3, Butandiol-1,4, Hexandiol-1,6, Decandiol-1,10, Dodecandiol-1,12, Glycerin, Diglycerin, Trimethylolpropan, Di-trimethylolpropan, Pentaerythrit und Dipentaerythrit gebildeten Gruppe bedeutet.

3. Verfahren nach Anspruch 1, dadurch gekennzeichnet, daß man die vicinal hydroxy, alkoxy- bzw. hydroxy, hydroxyalkylenoxy-substituierten $C_{16}$-$C_{22}$-Fettalkoholester der allgemeinen Formel IV bzw. IVa herstellt, indem man Fettalkohole der Formel $R^1$-OH, in der $R^1$ ein $C_{16}$-$C_{22}$-Alkenylrest mit einer olefinischen Doppelbindung oder mehreren ist, mit einem reaktiven Derivat einer $C_2$-$C_4$-Monocarbonsäure acyliert, die acylierten $C_{16}$-$C_{22}$-Fettalkohole epoxidiert und die Oxirangruppen der erhaltenen Epoxyfettalkoholester mit Monoalkanolen mit 1 bis 22 Kohlenstoffatomen bzw. mit mehrfunktionellen Alkanolen mit 2 bis 12 Kohlenstoffatomen und 2 bis 4 OH-Gruppen in Gegenwart katalytisch wirkender Lewissäuren umsetzt.

4. Verfahren nach Anspruch 3, dadurch gekennzeichnet, daß man die Epoxyfettalkoholester mit difunktionellen linearen Alkanolen mit 2 bis 12 Kohlenstoffatomen in Einsatzmolverhältnissen von etwa 2:1

umsetzt.

5. Verfahren nach Anspruch 3, dadurch gekennzeichnet, daß man die Epoxyfettsäureester mit den Monoalkanolen mit 1 bis 22 Kohlenstoffatomen bzw. mehrfunktionellen Alkanolen mit 2 bis 12 Kohlenstoffatomen und 2 bis 4 OH-Gruppen in Einsatzmolverhältnissen von etwa 1:1 bis 1:6 umsetzt.

Europäisches Patentamt

# EUROPÄISCHER RECHERCHENBERICHT

Nummer der Anmeldung

EP 89 11 5540

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | US-A-2 491 533 (D. SWERN)<br>* Insgesamt * | 1-5 | C 07 C 43/13<br>C 07 C 69/02<br>C 07 D 303/16<br>C 07 C 41/26 |
| A | GB-A- 741 492 (HENKEL)<br>* Ansprüche; Beispiele 1,2,6 * | 1-5 | |
| A | EP-A-0 113 798 (HENKEL)<br>* Ansprüche; Seiten 12,13 * | 1-5 | |
| A | FR-A-2 058 053 (DEUTSCHE GOLD- UND SILBER-SCHEIDEANSTALT)<br>* Seite 1, Zeile 1 - Seite 2, Zeile 15 * | 1-5 | |

RECHERCHIERTE SACHGEBIETE (Int. Cl.5)

C 07 C 41/00
C 07 C 43/00
C 07 C 69/00

Der vorliegende Recherchenbericht wurde für alle Patentansprüche erstellt

| Recherchenort | Abschlußdatum der Recherche | Prüfer |
|---|---|---|
| DEN HAAG | 07-12-1989 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)